# FASCICULE DE BREVET EUROPEEN

(11) **EP 1 426 100 B1**
(45) Date de publication et mention de la délivrance du brevet: **14.07.2010**
(21) Numéro de dépôt: 03292929.1
(22) Date de dépôt: 26.11.2003
(51) Int. Cl.: B01J 13/04, A61K 9/14, A61K 8/11

(54) **Stabilisation de principes actifs aromatiques par des polymères aromatiques**
Stabilisieren von aromatischen Komponenten durch aromatische Polymere
Stabilisation of aromatic moieties by aromatic polymers

(30) Priorité: 04.12.2002 FR 0215297
(43) Date de publication de la demande: 09.06.2004
(73) Titulaire: L'Oréal, 75008 Paris (FR)
(72) Inventeur: Toumi, Leila, 95110 Sannois (FR); Bernard, Anne-Laure, 299949 Singapour (FR); Samain, Henri, 91570 Bievres (FR); Nicolas-Morgantini, Luc, 60810 Rully (FR)
(74) Mandataire: Casalonga, Axel

(56) Documents cités:
- WO-A-01/51018
- US-A- 4 533 617
- US-A- 5 213 812
- US-B1- 6 416 799

## Description

La présente invention concerne un procédé de protection et/ou de stabilisation de principes actifs aromatiques par incorporation ou encapsulation d'un tel principe actif dans un polymère synthétique à groupes aryle, des particules obtenues par un tel procédé et des compositions, notamment des compositions cosmétiques, contenant de telles particules.

La stabilisation de principes actifs hydrosolubles de faible masse dans des compositions pharmaceutiques ou cosmétiques contenant une certaine fraction d'eau est un problème récurrent. L'encapsulation des principes actifs dans des microcapsules ou nanocapsules classiques ― c'est-à-dire des particules constituées d'une phase liquide hydrophile entourée d'une enveloppe polymérique ― obtenues soit par polymérisation interfaciale, soit par émulsion multiple eau/huile/eau suivie d'une évaporation du solvant, soit par coacervation, s'est avérée dans la plupart des cas insatisfaisante car les capsules préparées relarguent généralement les principes actifs plus ou moins vite dans la phase aqueuse extérieure. Un tel relargage est également observé pour d'autres types de structures organisées tels que des vésicules.

L'incorporation dans des particules polymériques solides est également problématique car les principes actifs hydrosolubles sont généralement incompatibles avec la matrice polymérique et l'on observe un "suintement" c'est-à-dire une lente migration des molécules de principe actif vers le milieu extérieur.

La demanderesse a résolu ces problèmes de relargage pour un groupe particulier de principes actifs de faible masse, à savoir pour des principes actifs comportant un ou plusieurs noyaux aromatiques, en incorporant ces principes actifs dans des particules comprenant un polymère qui porte un certain nombre de groupes aryle et qui, à température ambiante, est à l'état vitreux.

Les principes actifs aromatiques enfermés dans une telle matrice ou enveloppe de polymère arylé vitreux, ne sont par relargués dans la phase aqueuse environnante et se trouvent parfaitement protégés contre des agents chimiques ou physico-chimiques susceptibles d'entraîner leur dégradation.

La présente invention a par conséquent pour objet des particules comprenant
- au moins un principe actif, de préférence hydrosoluble, comportant un ou plusieurs groupes aromatiques, carbocycliques ou hétérocycliques, monocycliques ou polycycliques condensés, ayant un poids moléculaire inférieur ou égal à 1000, et
- au moins un polymère synthétique à groupes aryle ayant une température de transition vitreuse (T_{g}) supérieure ou égale à 45 °C.

De préférence les groupements aryle du polymère synthétique sont des groupements phényle ou phénylène.

Lorsque l'actif est de nature hétérocyclique, il s'agit de préférence d'une structure polycyclique comportant au moins un noyau benzénique.

Elle a encore pour objet un procédé de préparation de telles particules, autrement dit un procédé de protection ou de stabilisation de principes actifs aromatiques de faible masse par incorporation d'un tel principe actif dans une matrice d'un polymère arylé, ou par enrobage ou encapsulation d'un tel principe actif hydrosoluble aromatique dans une enveloppe formée d'un tel polymère arylé.

Enfin, la présente invention a pour objet une composition, en particulier une composition cosmétique, contenant les particules décrites ci-dessus.

Les principes actifs stabilisés par incorporation dans une matrice ou enveloppe polymérique arylée selon l'invention sont choisis parmi les principes actifs hydrosolubles aromatiques de faible poids moléculaire c'est-à-dire inférieur à 1000 et de préférence inférieur à 500. Ces principes actifs sont de préférence hydrosolubles.

On entend par principe actif hydrosoluble selon l'invention un principe actif ayant une solubilité dans l'eau, mesurée à 25 °C, au moins égale à 0,1 g/l (obtention d'une solution macroscopiquement isotrope et transparente, colorée ou non). Cette solubilité est de préférence supérieure ou égale à 1 g/l.

Le caractère hydrophile des principes actifs aromatiques peut être apporté par un ou plusieurs substituants ionisables ou ionisés, tels que des groupes sulfonate, sulfate, carboxylate, phosphonate, phosphate, amine primaire, secondaire ou tertiaire ou un groupe ammonium.

Les principes actifs sont de préférence des principes actifs cosmétiques.

On peut citer à titre d'exemples de tels principes actifs cosmétiques
- les colorants capillaires tels que les colorants directs et les précurseurs de colorants d'oxydation,
- les filtres UV-A et UVB,
- les vitamines comportant un cycle aromatique telles que la vitamine B12,
- les flavonoïdes que l'on trouve dans les extraits de seigle,
- les agents réducteurs aromatiques.

Le terme "polymères synthétiques à groupes aryle" utilisé pour désigner les polymères servant à la fabrication des particules de la présente invention englobe des polymères comportant un certain nombre de groupes aryle et/ou arylène, de préférence phényle et phénylène, ces derniers pouvant former la chaîne principale du polymère ou bien faire partie des chaînes latérales. Il peut s'agir d'homopolymères ou de copolymères séquencés, statistiques ou alternés, obtenus par polyaddition ou par polycondensation. Ce terme englobe également les polymères de greffage ayant une structure ramifiée ou réticulée.

Les polymères arylés formant la matrice ou l'enveloppe des particules de la présente invention, doivent, bien entendu, être insolubles dans l'eau et dans la plupart des solvants cosmétiquement acceptables. Les polymères arylés utilisés dans la présente invention sont par conséquent de préférence non-ioniques, c'est-à-dire exempts de charges susceptibles de les rendre solubles dans l'eau ou dans d'autres solvants polaires.

Comme indiquée ci-dessus, la température de transition vitreuse du polymère formant la matrice ou l'enveloppe des particules selon l'invention doit au moins être égale à 45 °C. Elle est de préférence supérieure ou égale à 50 °C et idéalement supérieure ou égale à 60 °C. Ce paramètre est essentiel pour l'invention. En effet, il s'est avéré que le caractère figé, vitreux du polymère à température ambiante était une condition indispensable pour empêcher la migration des molécules de principe actif encapsulées vers la surface des particules ou bien la pénétration d'agents chimiques à l'intérieur des particules. Or, les particules de la présente invention et les compositions les contenant sont susceptibles d'être exposés à des températures relativement élevées qui règnent par exemple l'été à la plage. Lorsque la température des particules s'approche alors de la température de transition vitreuse du polymère, celui-ci se plastifie et il y a un risque de fuite du principe actif hydrophile vers l'extérieur des particules.

Un groupe de polymères arylés préférés selon l'invention est formé par les polyorganosiloxanes comportant des groupes aryle qui sont portés directement par les atomes de silicium du squelette siloxane. Les polyorganosiloxanes arylés peuvent avoir une structure linéaire, ramifiée ou réticulée.

La teneur appropriée en groupes aryle, et en particulier phényle, des polyorganosiloxanes dépend, entre autres, de la quantité de principe actif à incorporer. En effet, la demanderesse a constaté que pour un type de polymère donné, la quantité de principe actif qu'il est possible d'encapsuler de manière stable augmente avec la teneur en groupes aryle. Par ailleurs, la teneur en groupes aryle a une influence sur la température de transition vitreuse, celle-ci étant généralement plus élevée pour des teneurs en groupes aryle plus importantes.

La demanderesse a obtenu des résultats satisfaisants pour une teneur en groupes aryle telle que le rapport du nombre de groupes aryle au nombre d'atomes de silicium est comprise entre 1/15 et 2/1. On préfère en particulier des polyorganosiloxanes ayant un rapport du nombre de groupes aryle au nombre d'atomes de silicium compris dans l'intervalle allant de 1/10 à 2/1.

On peut citer à titre d'exemple de tels polyorganosiloxanes phénylés le polymère DC(R)Z-6018 commercialisé par la société DOW CORNING.

Une autre famille de polymères arylés utilisables pour la stabilisation de principes actifs aromatiques hydrosolubles englobe les polycondensats obtenus à partir de monomères arylés.

Ces polycondensats sont par exemple des polyuréthanes obtenus par polycondensation d'au moins un diisocyanate et d'au moins un composé comportant deux fonctions à hydrogène labile, choisies parmi les fonctions hydroxyle, thiol, amine primaire et amine secondaire, les monomères étant choisis de manière à ce qu'au moins un type de comonomères comporte un groupe aryle.

Il ressort de ce qui précède que le terme "polyuréthanes", tels qu'il est utilisé dans la présente demande, englobe non seulement les polyuréthanes proprement dits comportant des liaisons carbamate (-NH-CO-O), mais également les polyurées (à liaisons -NH-CO-NH- ou -NR-CO-NH-) et les polythiourées (à liaisons -NH-CO-S-).

Les polycondensats arylés peuvent également être des polyesters ou polyamides obtenus par polycondensation d'au moins un diacide ou d'un dérivé activé d'un diacide, avec respectivement au moins un diol ou au moins une diamine, ces monomères étant choisis, comme précédemment, de manière à ce qu'au moins un des types de monomères comporte un groupe aryle.

Les monomères donnant les polycondensats arylés ci-dessus, à savoir les polyuréthannes, polyesters, polyamides et copolymères de ceux-ci, sont connus dans la technique.

On peut citer à titre d'exemple de monomères non-arylés :
- les diisocyanates aliphatique tels que l'hexaméthylènediisocyanate et l'isophorone-diisocyanate,
- les diols aliphatiques tels que les alkylèneglycols ou polyalkylèneglycols,
- les diamines aliphatiques telles que les alkylènediamines,
- les diacides aliphatique tels que l'acide succinique, l'acide fumarique, l'acide adipique et l'acide sébacique, ou les anhydrides ou chlorures de ces diacides,
- les aminoalcools,
- les aminoacides.

A titre d'exemples de monomères arylés, on peut citer
- les diisocyanates aromatiques tels que le toluènediisocyanate et le dihénylméthanediisocyanate,
- les diamines aromatiques telles que les phénylènediamines, éventuellement substituées,
- les diols aromatiques tels que les bisphénols, par exemple le bisphénol A,
- les diacides aromatiques tels que l'acide téréphtalique et l'acide isophtalique.

La polycondensation des monomères divalents énumérés ci-dessus aboutit à des structures linéaires. Pour l'obtention de structures ramifiées, on peut ajouter une certaine fraction de monomères au moins trivalents, tels que le trichlorure d'acide 1,3,5-benzènetricarboxylique, le tétraisocyanate de silicium, la 2,4,6-triamino-1,3,5-triazine, le glycérol, le polyglycérol, ou le glycérol polypropoxylé à terminaisons amino.

Dans un mode de réalisation préféré de l'invention, au moins un type de monomères formant les polycondensats arylés mentionnés ci-dessus (polyuréthanes, polyesters et polyamides) comporte une liaison S-S. Ce monomère peut être différent de celui ou de ceux comportant un groupe aryle ou peut être identique à celui-ci, autrement dit il peut comporter à la fois un groupe aryle et une liaison S-S.

A titre d'exemples de monomères comportant une liaison S-S ou à la fois un groupe aryle et une liaison S-S on peut citer, par exemple,
- le bis-4-aminophényldisulfure, l'homocystine, la cystamine, le formamidine-disulfure, le di-(2-hydroxyéthyl)disulfure.

Les polymères à liaisons S-S sont particulièrement intéressants dans les cas où l'utilisation du principe actif nécessite la libération de celui-ci dans le milieu environnant. Une telle libération peut alors se faire par coupure des liaisons S-S des polymères arylés, à l'aide d'un agent chimique approprié.

Les particules de la présente invention peuvent avoir des structures et des tailles très variables découlant de leur procédé de préparation et liées à l'application envisagée.

Il peut s'agir par exemple de microparticules obtenues par broyage d'un matériau solide constitué d'une matrice de polymère arylé dans laquelle sont finement dispersés ou dissous (solution solide) le ou les principe(s) actif(s) aromatique(s). Ces microparticules ont de préférence une taille moyenne comprise entre 0,5 et 500 µm.

D'autres microparticules ayant une taille comprise entre 0,05 et 500 µm peuvent être obtenues par émulsion multiple. Elles ont une structure de type noyau-enveloppe dans laquelle une enveloppe formée par le polymère arylé entoure hermétiquement un noyau liquide contenant le principe actif aromatique hydrophile. La technique de l'émulsion multiple utilisée pour la formation de ce type de particules est décrite par exemple dans FR 2 766 737 et dans DE 19719297.

Dans un autre mode de réalisation, les particules sont des particules à structure noyau-enveloppe ayant une taille relativement plus importante, comprise de préférence entre 0,5 et 10 mm. Ces particules sont obtenues par coextrusion de deux phases, la phase extérieure étant formée par le polymère arylé, à l'état fondu ou sous forme de solution visqueuse, de préférence de viscosité (mesurée à 25 °C) supérieure à 1000 centipoises et la phase interne contenant le principe actif à l'état fondu ou sous forme de solution épaisse.

Pour protéger efficacement le principe actif dans la matrice polymérique des particules de l'invention, il est préférable que le rapport en poids du principe actif au polymère arylé ne dépasse une certaine valeur limite qui dépend par exemple du taux de groupes aryle du polymère. Plus celui-ci est important, plus la limite supérieure du rapport en poids principe actif/polymère arylé donnant lieu à une protection efficace est élevée. La demanderesse a constaté que l'on obtient généralement des résultats d'encapsulation satisfaisants pour un rapport en poids du principe actif hydrosoluble aromatique au polymère arylé compris entre 1/1 et 1/50, de préférence entre 1/3 et 1/20.

Comme indiqué en introduction, la présente invention a également pour objet un procédé de protection ou de stabilisation de principes actifs aromatiques. Ce procédé comprend plus précisément l'incorporation d'un principe actif, de préférence hydrosoluble, comportant un ou plusieurs groupes aromatiques, carbocycliques ou hétérocycliques, monocycliques ou polycycliques condensés, et ayant un poids moléculaire inférieur ou égal à 1000, dans une matrice d'un polymère synthétique à groupes aryle ayant une température de transition vitreuse (T_{g}) supérieure ou égale à 45 °C, ou l'enrobage ou l'encapsulation d'un tel principe actif dans une enveloppe formée d'un tel polymère à groupes aryle.

L'incorporation du principe actif dans une matrice peut se faire soit par fusion soit par dissolution du polymère et du principe actif.

Le procédé par fusion comprend les étapes suivantes consistant
- à faire fondre le polymère synthétique à groupes aryle,
- à dissoudre ou à disperser finement le principe actif aromatique dans le polymère fondu,
- à laisser refroidir le mélange ainsi obtenu jusqu'à température ambiante, et
- à broyer le matériau solide jusqu'à une taille de particules appropriée, de préférence comprise entre 0,05 et 500 µm.

Ce procédé présente l'avantage de pouvoir se faire en l'absence de solvants organiques. Il présente toutefois la difficulté de ne pouvoir être mis en oeuvre qu'avec des polymères ayant une température de fusion inférieure à la température de décomposition du principe actif.

Le procédé par dissolution permet de remédier à cet inconvénient. Ce procédé comprend les étapes suivantes consistant
- à dissoudre le polymère synthétique à groupes aryle et le principe actif aromatique dans un solvant ou mélange de solvants approprié,
- à évaporer le solvant de manière à obtenir un matériau solide, et
- à broyer le matériau solide jusqu'à une taille de particules appropriée, de préférence comprise entre 0,05 et 500 µm.

Ce procédé nécessite la sélection d'un solvant ou mélange de solvants permettant de dissoudre à la fois le polymère aryle hydrophobe et le principe actif qui est souvent hydrophile.

On peut citer à titre d'exemples de tels solvants ou mélanges de solvants les alcools en C₁₋₄ tels que le méthanol, l'éthanol, l'isopropanol. On peut utiliser aussi d'autres solvants organiques tels que le DMSO et le THF.

Le broyage du matériau solide vitreux obtenu après refroidissement à température ambiante ou après évaporation du solvant, peut se faire à l'aide de n'importe quel type de broyeur connu dans la technique permettant d'obtenir des particules ayant la taille souhaitée, de préférence comprise entre 0,05 et 500 µm. On peut citer à titre d'exemples les broyeurs à couteaux, les broyeurs à billes, les broyeurs à broches.

Enfin, selon un troisième mode de réalisation, le procédé de protection ou de stabilisation de principes actifs hydrosolubles aromatiques comprend la coextrusion du principe actif hydrosoluble aromatique et du polymère synthétique à groupes aryle de manière à obtenir des particules constituées d'un noyau solide formé par le principe actif hydrosoluble aromatique, et d'une enveloppe solide formée par le polymère synthétique à groupes aryle.

La coextrusion du polymère arylé et du principe actif hydrosoluble aromatique se fait selon un procédé connu décrit par exemple dans Harper J.M., 1990, *Extrusion of Foods in biotechnology and food process engineering,* éditeur Marcel DEKER Inc., chapitre 10, page 307.

La présente invention a encore pour objet des compositions, de préférence des compositions cosmétiques, contenant, dans un milieu cosmétiquement ou physiologiquement acceptable, les particules décrites ci-dessus. La quantité de particules présente dans les compositions de la présente invention dépend de la taille des particules, de leur teneur en principe actif et bien entendu du type de formulation et de l'application envisagée pour celle-ci. De manière générale, les compositions de la présente invention contiennent de 0,1 à 95 % en poids, de préférence de 1 à 50 % en poids de particules rapporté au poids total de la composition.

Le milieu cosmétiquement ou physiologiquement acceptable doit être inerte vis-à-vis du polymère aryle utilisé pour la préparation des particules, et en particulier ne doit ni dissoudre ni gonfler ce polymère.

Le milieu cosmétiquement ou physiologiquement acceptable contient de préférence une importante fraction d'eau et il convient de veiller à ce que la concentration de certains solvants organiques susceptibles de dissoudre le polymère, tels que l'éthanol ou les glycols ne dépasse pas une certaine valeur.

Les compositions selon la présente invention peuvent contenir en outre toutes sortes de principes actifs et d'ingrédients de formulation utilisés habituellement dans le domaine cosmétique.

Les principes actifs cosmétiques peuvent être choisis parmi les vitamines ou provitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les acides et alcools gras ramifiés ou non, les esters gras, les cires animales, végétales ou minérales, les huiles végétales, minérales ou synthétiques, les céramides et les pseudo-céramides, les acides organiques hydroxylés, les filtres UV, les agents antipelliculaires, les agents régulateurs de séborrhée, les agents apaisants, les agents tensioactifs cationiques, les polymères cationiques, les polymères amphotères, les silicones organomodifiées ou non, les polymères associatifs de type non-ionique, cationique ou amphotère comportant au moins une chaîne grasse.

Les adjuvants de formulation sont choisis par exemple parmi les agents épaississants, les agents d'ajustement et de fixation du pH, les agents conservateurs, les séquestrants, les opacifiants, les agents réducteurs ou antioxydants, les parfums et les agents tensioactifs non-cationiques.

Bien entendu, l'homme du métier veillera à choisir le ou les éventuels composés complémentaires ci-dessus de manière à ce que les propriétés avantageuses intrinsèques des compositions selon l'invention ne soient pas altérées par la ou les adjonctions envisagées.

Les compositions de la présente invention sont de préférence des compositions de teinture des fibres kératiniques, et les principes actifs stabilisés sont alors choisis de préférence parmi les colorants capillaires.

Dans un autre mode de réalisation, les compositions de la présente invention sont des compositions solaires et les principes actifs encapsulés sont alors des filtres UV organiques.

Dans encore un autre mode de réalisation, les compositions de la présente invention sont des compositions autobronzantes et les principes actifs encapsulés sont des actifs autobronzants hydrophiles tels que l'apigénidine.

Les exemples ci-après mettent en évidence en particulier l'effet protecteur de l'encapsulation des principes actifs hydrophiles aromatiques dans une matrice ou enveloppe en polymère arylé.

### Exemple 1

### Préparation de particules broyées

On chauffe un phénylpropylsilsesquioxane de masse 1500 - 2500 g/mole, commercialisé soùs la dénomination DC® Z-6018, par la société Dow Corning, (T_{g} 48 °C) à une température de 130 °C de manière à le faire fondre, et l'on y disperse jusqu'à homogénéité 10 % en poids, rapporté au polysiloxane, de colorant azoïque (MIP RED 2985-D, société Ciba). Après refroidissement à température ambiante, on broie le matériau solide, vitreux obtenu à l'aide d'un mortier jusqu'à une taille moyenne de particules d'environ 500 µm, on lave la poudre obtenue trois fois avec de l'eau ou avec une solution aqueuse d'un agent de dispersion (diméthicone copolyol isostéarate) sous agitation forte pendant 24 heures avec une secoueuse (Prolabo). Ce lavage a pour objectif d'éliminer les molécules de colorant qui se retrouvent à la surface des particules broyées. Un dosage de la quantité de colorant contenue dans les particules ainsi lavées révèle que 8 % du colorant ont été éliminés par lavage, c'est-à-dire le taux d'encapsulation est de 92 %.

La poudre est ensuite dispersée, à l'aide du dispersant siliconé ci-dessus, dans une solution ammoniacale à pH 10,5 contenant 0,53 % en poids de métabisulfite de sodium, et dans une solution ammoniacale à pH 10,5 exempt d'agent réducteur.

Après 2 mois de conservation de ces deux suspensions à une température de 45 °C, le dosage de la quantité de colorant ayant fui dans le milieu aqueux ammoniacal exempt de métabisulfite indique un taux d'encapsulation de 90 %, au lieu des 92 % mesurés initialement, c'est-à-dire une perte d'environ 2 % de la quantité de colorant initialement encapsulée.

Le dosage de la quantité de colorant restant dans les particules conservées pendant 2 mois à 45 °C dans la solution de métabisulfite de sodium, par spectrométrie UV-visible d'une solution de la poudre dans un mélange chloroforme/méthanol (9/1), révèle une perte de colorant d'environ 5 % (87 % de colorant restant au lieu des 92 % initialement encapsulés).

Le fait que la perte de colorant en présence d'agent réducteur (5 %) est légèrement supérieure à celle observée en l'absence d'agent réducteur (2 %) montre qu'une faible fraction de colorant encapsulé a été détruite par l'agent réducteur. Ce résultat peut être attribué à la très grande surface spécifique de la poudre finement dispersée qui favorise les échanges entre les particules et le milieu environnant.

### Exemple 2

### Encapsulation d'extrait de sorgho

Le troisième exemple porte sur l'encapsulation d'extrait de sorgho, matière colorante complexe utilisée notamment pour la coloration de la peau dans des compositions autobronzantes. Or, la plupart des compositions autobronzantes contiennent, comme principal principe actif autobronzant, de la dihydroxyacétone (DHA) capable de dégrader chimiquement l'apigénidine qui est un colorant présent dans l'extrait de sorgho. Il s'agit donc de protéger l'extrait de sorgho, et en particulier l'apigénidine, contre la dégradation par la dihydroxyacétone.

L'encapsulation de l'extrait de sorgho se fait en milieu solvant. On solubilise 1 partie d'extrait de sorgho et 10 parties de phénylpropylsilsesquioxane (DC(R) Z-6018, Dow Corning) dans une quantité suffisante d'éthanol. Après solubilisation complète, on évapore l'éthanol sous vide et on broie le résidu solide au mortier jusqu'à une taille moyenne des particules d'environ 500 µm. On lave ces particules de la manière décrite dans l'exemple 2 dans une solution aqueuse.

Pour évaluer le taux d'encapsulation après lavage, on dose par spectrométrie UV-visible l'apigénidine après dissolution de la poudre dans du méthanol acide (contenant 0,1 % en volume d'HCl à 36 %). Le taux d'encapsulation après lavage mesuré ainsi est compris entre 90 et 95 %.

Pour évaluer l'efficacité de protection de l'extrait de sorgho contre la dégradation chimique par la DHA, on prépare une suspension aqueuse, gélifiée par de l'hydroxypropylcellulose, contenant 2 % de poudre et 4 % de DHA, ainsi qu'une suspension aqueuse, gélifiée par de l'hydroxypropylcellulose, contenant 0,2 % d'extrait de sorgho (non encapsulé) et 4 % de DHA.

Après 1 mois de conservation de ces deux échantillons à température ambiante, le taux de dégradation de l'extrait de sorgho est de 10 % en poids pour l'échantillon encapsulé et de 35 % en poids pour l'échantillon non encapsulé.

## Revendications

1. Particules comprenant
• au moins un principe actif comportant un ou plusieurs groupes aromatiques, carbocycliques ou hétérocycliques, monocycliques ou polycycliques condensés, ayant un poids moléculaire inférieur ou égal à 1000, et
• au moins un polymère synthétique à groupes aryle ayant une température de transition vitreuse T_{g} supérieure ou égale à 45 °C.

2. Particules selon la revendication 1, **caractérisées par le fait que** le ou les principes actifs sont hydrosolubles.

3. Particules selon la revendication 1 ou 2, **caractérisées par le fait que** les groupes aryle du polymère synthétique sont des groupes phényle ou phénylène.

4. Particules selon l'une quelconque des revendications précédentes, **caractérisées par le fait que** le principe actif aromatique a un poids moléculaire inférieur ou égal à 500.

5. Particules selon l'une quelconque des revendications précédentes, **caractérisées par le fait que** le principe actif aromatique porte au moins un groupe ionisé ou ionisable.

6. Particules selon l'une quelconque des revendications précédentes, **caractérisées par le fait que** le rapport en poids du principe actif au polymère aryle est compris entre 1/1 et 1/50, de préférence entre 1/3 et 1/20.

7. Particules selon l'une quelconque des revendications précédentes, **caractérisées par le fait que** le principe actif aromatique est un principe actif cosmétique.

8. Particules selon la revendication 5, **caractérisées par le fait que** le principe actif cosmétique est un colorant capillaire, un filtre UV organique ou un flavonoïde.

9. Particules selon l'une quelconque des revendications précédentes, **caractérisées par le fait que** le polymère synthétique à groupes aryle est un polymère non-ionique.

10. Particules selon l'une quelconque des revendications précédentes, **caractérisées par le fait que** le polymère à groupes aryle est un polyorganosiloxane comportant des groupes aryle liés directement aux atomes de silicium du squelette siloxane.

11. Particules selon la revendication 10, **caractérisées par le fait que** le rapport du nombre de groupes aryle au nombre d'atomes de silicium du polymère est compris entre 1/15 et 2/1.

12. Particules selon l'une quelconque des revendications 1 à 9, **caractérisées par le fait que** le polymère synthétique à groupes phényle est un polyuréthane obtenu par polycondensation
• d'au moins un diisocyanate et
• d'au moins un composé comportant deux fonctions à hydrogène labile, choisies parmi les fonctions hydroxyle, thiol, amine primaire et amine secondaire,
les monomères étant choisis de manière à ce qu'au moins un type de monomères comporte un groupe aryle.

13. Particules selon l'une quelconque des revendications 1 à 9, **caractérisées par le fait que** le polymère synthétique à groupes aryle est un polyester ou polyamide obtenu par polycondensation
• d'au moins un diacide ou d'un dérivé activé d'un diacide, et
• respectivement d'au moins un diol ou d'au moins une diamine,
les monomères étant choisis de manière à ce qu'au moins un type de monomères comporte un groupe phényle.

14. Particules selon la revendication 12 ou 13, **caractérisées par le fait qu'**au moins un type de monomères comporte une liaison S-S.

15. Particules selon l'une quelconque des revendications précédentes, **caractérisées par le fait qu'**il s'agit de microparticules ayant une taille moyenne comprise entre 0,05 et 500 µm, obtenues par broyage d'un matériau solide.

16. Particules selon l'une quelconque des revendications 1 à 14, **caractérisées par le fait qu'**il s'agit de microcapsules à structure noyau-enveloppe ayant une taille moyenne comprise entre 0,05 et 500 µm, obtenues par émulsion multiple.

17. Particules selon l'une quelconque des revendications 1 à 14, **caractérisées par le fait qu'**il s'agit de particules enrobées ayant une taille moyenne comprise entre 0,5 et 10 mm, obtenues par coextrusion.

18. Procédé de protection ou de stabilisation de principes actifs aromatiques, de préférence hydrosolubles, comprenant l'incorporation d'un principe actif hydrosoluble comportant un ou plusieurs groupes aromatiques, carbocycliques ou hétérocycliques, monocycliques ou polycycliques condensés, et ayant un poids moléculaire inférieur ou égal à 1000, dans une matrice d'un polymère synthétique à groupes aryle ayant une température de transition vitreuse T_{g} supérieure ou égale à 45°C, ou l'enrobage ou l'encapsulation d'un tel principe actif hydrosoluble aromatique dans une enveloppe formée d'un tel polymère à groupes aryle.

19. Procédé selon la revendication 18, **caractérisé par le fait qu'**il comprend les étapes suivantes consistant
• à faire fondre le polymère synthétique à groupes aryle,
• à dissoudre ou à disperser finement le principe actif aromatique dans le polymère fondu,
• à laisser refroidir le mélange ainsi obtenu jusqu'à température ambiante, et
• à broyer le matériau solide jusqu'à une taille de particules appropriée.

20. Procédé selon la revendication 18, **caractérisé par le fait qu'**il comprend les étapes suivantes consistant
• à dissoudre le polymère synthétique à groupes aryle et le principe actif aromatique dans un solvant ou mélange de solvants approprié,
• à évaporer le solvant de manière à obtenir un matériau solide, et
• à broyer le matériau solide jusqu'à une taille de particules appropriée.

21. Procédé selon la revendication 18, **caractérisé par le fait qu'**il comprend une étape de formation d'une émulsion multiple.

22. Procédé selon la revendication 18, **caractérisé par le fait que** l'on coextrude le principe actif aromatique et le polymère synthétique à groupes aryle de manière à obtenir des particules constituées d'un noyau solide formé par le principe actif hydrosoluble aromatique, et d'une enveloppe solide formée par le polymère synthétique à groupes aryle.

23. Composition contenant, dans un milieu cosmétiquement ou physiologiquement acceptable, des particules selon l'une quelconque des revendications 1 à 17.

24. Composition selon la revendication 23, **caractérisée par le fait que** les particules représentent de 0,1 % à 95 % en poids, de préférence de 1 % à 50 % en poids, de la composition totale.

## Claims

1. Particles comprising:
• at least one active principle comprising one or more aromatic, carbocyclic or heterocyclic, monocyclic or fused polycyclic groups, with a molecular weight of less than or equal to 1000, and
• at least one synthetic polymer containing aryl groups, with a glass transition temperature T_{g} of greater than or equal to 45°C.

2. Particles according to Claim 1, **characterized in that** the active principle(s) is (are) water-soluble.

3. Particles according to Claim 1 or 2, **characterized in that** the aryl groups of the synthetic polymer are phenyl or phenylene groups.

4. Particles according to any one of the preceding claims, **characterized in that** the aromatic active principle has a molecular weight of less than or equal to 500.

5. Particles according to any one of the preceding claims, **characterized in that** the aromatic active principle bears at least one ionized or ionizable group.

6. Particles according to any one of the preceding claims, **characterized in that** the weight ratio of the active principle to the aryl-containing polymer is between 1/1 and 1/50 and preferably between 1/3 and 1/20.

7. Particles according to any one of the preceding claims, **characterized in that** the aromatic active principle is a cosmetic active principle.

8. Particles according to Claim 5, **characterized in that** the cosmetic active principle is a hair dye, an organic UV-screening agent or a flavonoid.

9. Particles according to any one of the preceding claims, **characterized in that** the synthetic polymer containing aryl groups is a nonionic polymer.

10. Particles according to any one of the preceding claims, **characterized in that** the polymer containing aryl groups is a polyorganosiloxane comprising aryl groups linked directly to the silicon atoms of the siloxane skeleton.

11. Particles according to Claim 10, **characterized in that** the ratio of the number of aryl groups to the number of silicon atoms in the polymer is between 1/15 and 2/1.

12. Particles according to any one of Claims 1 to 9, **characterized in that** the synthetic polymer containing phenyl groups is a polyurethane obtained by polycondensation of:
• at least one diisocyanate, and
• at least one compound comprising two functions containing labile hydrogen, chosen from hydroxyl, thiol, primary amine and secondary amine functions, the monomers being chosen such that at least one type of monomer comprises an aryl group.

13. Particles according to any one of Claims 1 to 9, **characterized in that** the synthetic polymer containing aryl groups is a polyester or polyamide obtained by polycondensation:
• of at least one diacid or of an activated derivative of a diacid, and
• respectively, of at least one diol or of at least one diamine,
the monomers being chosen such that at least one type of monomer comprises a phenyl group.

14. Particles according to Claim 12 or 13, **characterized in that** at least one type of monomer comprises an S-S bond.

15. Particles according to any one of the preceding claims, **characterized in that** they are microparticles with a mean size of between 0.05 and 500 µm, obtained by grinding a solid material.

16. Particles according to any one of Claims 1 to 14, **characterized in that** they are microcapsules of core-shell structure with a mean size of between 0.05 and 500 µm, obtained by multiple emulsion.

17. Particles according to any one of Claims 1 to 14, **characterized in that** they are coated particles with a mean size of between 0.5 and 10 mm, obtained by coextrusion.

18. Process for protecting or stabilizing aromatic active principles, which are preferably water-soluble, comprising the incorporation of a water-soluble active principle comprising one or more aromatic, carbocyclic or heterocyclic, monocyclic or fused polycyclic groups, with a molecular weight of less than or equal to 1000, into a matrix of a synthetic polymer containing aryl groups with a glass transition temperature Tg of greater than or equal to 45°C, or the coating or encapsulation of such an water-soluble aromatic active principle in a shell formed by such a polymer containing aryl groups.

19. Process according to Claim 18, **characterized in that** it comprises the following steps consisting in:
• melting the synthetic polymer containing aryl groups,
• dissolving or finely dispersing the aromatic active principle in the molten polymer,
• allowing the mixture thus obtained to cool to room temperature, and
• grinding the solid material to a suitable particle size.

20. Process according to Claim 18, **characterized in that** it comprises the following steps consisting in:
• dissolving the synthetic polymer containing aryl groups and the aromatic active principle in a suitable solvent or mixture of solvents,
• evaporating off the solvent so as to obtain a solid material, and
• grinding the solid material to a suitable particle size.

21. Process according to Claim 18, **characterized in that** it comprises a step of forming a multiple emulsion.

22. Process according to Claim 18, **characterized in that** the aromatic active principle and the synthetic polymer containing aryl groups are co-extruded so as to obtain particles consisting of a solid core formed by the aromatic water-soluble active principle, and of a solid shell formed by the synthetic polymer containing aryl groups.

23. Composition containing, in a cosmetically or physiologically acceptable medium, particles according to any one of Claims 1 to 17.

24. Composition according to Claim 23, **characterized in that** the particles represent from 0.1% to 95% by weight and preferably from 1% to 50% by weight of the total composition.

## Patentansprüche

1. Partikel, die umfassen:
- mindestens einen Wirkstoff, der eine oder mehrere aromatische, carbocyclische oder heterocyclische, monocyclische oder polycyclische kondensierte Gruppen aufweist, mit einer Molmasse von 1000 oder darunter, und
- mindestens ein synthetisches Polymer mit Arylgruppen, das eine Glasübergangstemperatur Tg von 45 °C oder darüber aufweist.

2. Partikel nach Anspruch 1, **dadurch gekennzeichnet, dass** der oder die Wirkstoff(e) wasserlöslich sind.

3. Partikel nach Anspruch 1 oder 2, **dadurch gekennzeichnet, dass** die Arylgruppen des synthetischen Polymers Phenyl oder Phenylen bedeuten.

4. Partikel nach einem der vorhergehenden Ansprüche, **dadurch gekennzeichnet, dass** der aromatische Wirkstoff eine Molmasse von höchstens 500 aufweist.

5. Partikel nach einem der vorhergehenden Ansprüche, **dadurch gekennzeichnet, dass** der aromatische Wirkstoff mindestens eine ionisierte oder ionisierbare Gruppe trägt.

6. Partikel nach einem der vorhergehenden Ansprüche, **dadurch gekennzeichnet, dass** das Gewichtsverhältnis des Wirkstoffes und des Arylpolymers im Bereich von 1/1 bis 1/50 und vorzugsweise im Bereich von 1/3 bis 1/20 liegt.

7. Partikel nach einem der vorhergehenden Ansprüche, **dadurch gekennzeichnet, dass** der aromatische Wirkstoff ein kosmetischer Wirkstoff ist.

8. Partikel nach Anspruch 5, **dadurch gekennzeichnet, dass** der kosmetische Wirkstoff ein Haarfärbemittel, ein organischer UV-Filter oder ein Flavonoid ist.

9. Partikel nach einem der vorhergehenden Ansprüche, **dadurch gekennzeichnet, dass** das synthetische Polymer mit Arylgruppen ein nichtionisches Polymer ist.

10. Partikel nach einem der vorhergehenden Ansprüche, **dadurch gekennzeichnet, dass** das Polymer mit Arylgruppen ein Polyorganosiloxan ist, das Arylgruppen aufweist, die direkt an Siliciumatome des Polysiloxangerüsts gebunden sind.

11. Partikel nach Anspruch 10, **dadurch gekennzeichnet, dass** das Verhältnis der Anzahl der Arylgruppen und der Anzahl der Siliciumatome des Polymers im Bereich von 1/15 bis 2/1 liegt.

12. Partikel nach einem der Ansprüche 1 bis 9, **dadurch gekennzeichnet, dass** das synthetische Polymer mit Phenylgruppen ein Polyurethan ist, das erhalten wird durch Polykondensation
• mindestens eines Diisocyanats und
• mindestens einer Verbindung, die zwei Funktionen mit beweglichem Wasserstoff aufweist, die unter den Funktionen Hydroxy, Thio, primäres Amin und sekundäres Amin ausgewählt sind,
wobei die Monomere so ausgewählt sind, dass mindestens ein Typ von Monomeren eine Arylgruppe aufweist.

13. Partikel nach einem der Ansprüche 1 bis 9, **dadurch gekennzeichnet, dass** das synthetische Polymer mit Arylgruppen ein Polyester oder Polyamid ist, das erhalten wird durch Polykondensation
• mindestens einer Disäure oder eines aktivierten Derivats einer Disäure, und
• mindestens eines Diols bzw. mindestens eines Diamins, wobei die Monomere so ausgewählt sind, dass mindestens ein Typ von Monomeren eine Phenylgruppe aufweist.

14. Partikel nach Anspruch 12 oder 13, **dadurch gekennzeichnet, dass** mindestens ein Typ von Monomeren eine Bindung S-S aufweist.

15. Partikel nach einem der vorhergehenden Ansprüche, **dadurch gekennzeichnet, dass** es sich um Mikropartikel mit einer mittleren Größe im Bereich von 0,05 bis 500 µm handelt, die durch Zerkleinern eines festen Materials erhalten werden.

16. Partikel nach einem der einem der Ansprüche 1 bis 14, **dadurch gekennzeichnet, dass** es sich um Mikrokapseln mit Kern-Schale-Struktur handelt, die eine mittleren Größe im Bereich von 0,05 bis 500 µm aufweisen und über eine multiple Emulsion erhalten werden.

17. Partikel nach einem der einem der Ansprüche 1 bis 14, **dadurch gekennzeichnet, dass** es sich um umhüllte Partikel handelt, die eine mittleren Größe im Bereich von 0,5 bis 10 mm aufweisen und durch Coextrusion erhalten werden.

18. Verfahren zum Schutz oder zur Stabilisierung von aromatischen, vorzugsweise wasserlöslichen Wirkstoffen, das umfasst, einen wasserlöslichen Wirkstoff, der eine oder mehrere aromatische, carbocyclische oder heterocyclische, monocyclische oder polycyclische kondensierte Gruppen aufweist und eine Molmasse von 1000 oder darunter besitzt, in eine Matrix aus einem synthetischen Polymer mit Arylgruppen einzubringen, das eine Glasübergangstemperatur Tg von 45 °C oder darüber aufweist, oder einen solchen aromatischen wasserlöslichen Wirkstoff in einem Umhüllung einzubringen, die aus einem solchen Polymer mit Arylgruppen gebildet wird.

19. Verfahren nach Anspruch 18, **dadurch gekennzeichnet, dass** es die folgenden Schritte umfasst, die darin bestehen:
• das synthetische Polymer mit Arylgruppen zu schmelzen,
• den aromatischen Wirkstoff in dem geschmolzenen Polymer zu lösen oder fein zu dispergieren,
• das auf diese Wiese erhaltene Gemisch bis auf Raumtemperatur abkühlen zu lassen, und
• das feste Material auf eine geeignete Partikelgröße zu zerkleinern.

20. Verfahren nach Anspruch 18, **dadurch gekennzeichnet, dass** es die folgenden Schritte umfasst, die darin bestehen:
• das synthetische Polymer mit Arylgruppen und den aromatischen Wirkstoff in einem geeigneten Lösemittel oder Lösemittelgemisch zu lösen,
• das Lösemittel zu verdampfen, um einen Feststoff zu erhalten, und
• das feste Material auf eine geeignete Partikelgröße zu zerkleinern.

21. Verfahren nach Anspruch 18, **dadurch gekennzeichnet, dass** es einen Schritt der Bildung einer multiplen Emulsion umfasst.

22. Verfahren nach Anspruch 18, **dadurch gekennzeichnet, dass** der aromatische Wirkstoff und das synthetische Polymer mit Arylgruppen so coextrudiert werden, dass Partikel erhalten werden, die aus einem festen Kern, der aus dem aromatischen Wirkstoff gebildet ist, und einer Hülle aufgebaut sind, die aus dem synthetischen Polymer mit Arylgruppen gebildet ist.

23. Zusammensetzung, die in einem kosmetisch oder physiologisch akzeptablen Medium Partikel nach einem der Ansprüche 1 bis 17 enthält.

24. Zusammensetzung nach Anspruch 23, **dadurch gekennzeichnet, dass** die Partikel 0,1 bis 95 Gew.-% und vorzugsweise 1 bis 50 Gew.-% der gesamten Zusammensetzung ausmachen.
